# EUROPEAN PATENT APPLICATION

(11) **EP 0 839 829 A2**
(43) Date of publication of application: **06.05.1998**
(21) Application number: 97306586.5
(22) Date of filing: 28.08.1997
(51) Int. Cl.: C07H 21/00

(54) **Universal solid support oligonucleotide reagents**

(30) Priority: 06.09.1996 US 714841
(71) Applicant: BECKMAN INSTRUMENTS, INC., Fullerton California 92634-3100 (US)
(72) Inventor: Reddy,M. Parameswara, Brea, CA 92821 (US); Farooqui, Firdous, Brea, CA 92821 (US); Michael, Maged A., Placentia, CA 92870 (US); Zhong, Zhandong, Diamond Bar, CA 91765 (US)
(74) Representative: Ede, Eric

(57) **Abstract**

Oligonucleotide synthesis reagent having the following general formula are provided: 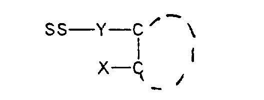 wherein SS is a solid support; Y is selected from the group consisting of esters, amides and thiol esters; X is selected from the group consisting of OH, SH, NH₂, and removable hydrogen protecting groups; and the dotted lines represent an aromatic ring system having at least one aromatic ring, X and Y being vicinally positioned at adjacent carbon atoms of the at least one aromatic right . The reagents are suitable for the efficient step-wise oligonucleotide synthesis and the subsequent fast cleavage and deprotection of the synthesized oligonucleotide.

## Description

### BACKGROUND OF THE INVENTION

### Field of the Invention

The present invention relates generally to oligonucleotide synthesis. More particularly, the present invention involves solid support reagents and methods for their use in the synthesis of oligonucleotides (e.g., DNA and RNA sequences).

### Description of Related Art

A variety of synthetic approaches have been developed for the preparation of oligonucleotide sequences. Typically, oligonucleotides are synthesized utilizing a building block approach which involves the sequential addition of nucleotides onto a growing oligonucleotide chain immobilized on to a solid support. Because every DNA oligonucleotide may have any of 4 different initial nucleotides, it is necessary to maintain a supply of 4 different nucleoside (A, C, G and T) loaded solid supports to be able to synthesize any given DNA sequence. In the case of DNA synthesis, the first nucleoside from the 3' end of the DNA sequence is typically preloaded on the solid support through an ester linkage. For example, if the sequence that is to be synthesized contains an initial T nucleoside, a T support is employed and the balance of the nucleotides in the DNA sequence added thereto (for example, using an automated DNA synthesizer). At the end of the total DNA synthesis, the oligonucleotide is cleaved from the solid support through the hydrolysis of the ester linkage. Taking into consideration RNA synthesis procedures, an additional 4-different nucleoside loaded solid supports must be available to the user. Similar considerations apply if any specialty modified nucleoside is desired at the 3' end or the 5' end if synthesis is carried out in the 5' to 3' direction.

Maintaining a supply of at least 8 different prederivatized solid supports is inconvenient and expensive. An additional consideration is the relatively short shelf life of nucleoside derivatized solid supports. Typically, after one year storage such solid supports are no longer usable. There is also the possibility that synthetic procedures may be initiated mistakenly with the wrong support leading to disastrous consequences in the final applications of the erroneously synthesized oligonucleotides.

In order to alleviate these problems some researchers have pursued developing some type of universal solid support. For example, deBear et al. derivatized glass supports with 2'(3')-O-benzoyluridine 5'-O-succinyl so that the uridine moiety is linked to the glass via an ester (succinate) linkage. [de Bear et al., Nucleosides and Nucleotides 6, 821-830 (1987)]. Oligonucleotide synthesis takes place by adding nucleotide monomers to the 2' or 3' position of the uridine. Following the synthesis, the new oligonucleotides can be released from the glass, deprotected and cleaved from the uridylyl terminus in one reaction.

Crea and Horn suggested a similar approach which involved preparing the dimer 5'-O-p-chlorophenylphospho-2'(3')-O-acetyluridilyl-[2'(3')-3']-5'-O-dimethoxytritylthymidine p-chlorophenylester and attaching the dimer to cellulose via a phosphate linkage. The 5' position of the thymidine is available for oligonucleotide attachment and synthesis. [R. Crea & T. Horn, Nucleic Acids Research 8, 2331 (1980)]. The subsequent use of aqueous concentrated ammonia results in the release of the synthesized oligonucleotide from the cellulose. Although Crea and Horn utilized the reactive vicinal groups on the uridine as the release site for the oligonucleotide from the uridine the solid support suggested in this reference is not a universal solid support since the initial nucleotide is incorporated in the solid support reagent and a different support is required for oligonucleotides incorporating different first nucleoside.

More recently, Schwartz et al. attached an adapter, 2'(3')-O-dimethoxytrityl-3'(2')-O-benzoyluridine-5'-O-(2-cyanoethyl N,N-diisopropylphosphoramidite, to a thymidine derivatized polystyrene and synthesized an oligonucleotide from the O-dimethoxytrityl position of the uridine. [M. E. Schwartz, R.R. Breaker, G.T. Asteriadis, and G.R. Gough, Tetrahedron Letters, Vol. 36, No. 1, pp 27 - 30, 1995] While this approach provides a universal solid support for oligonucleotide synthesis, the cleaving step releases the adapter and the thymidine from the support and then cleaves the synthesized oligonucleotide from the uridine.

The aforementioned solid supports and methods for their use disadvantages in terms of the convenience and efficiency of the subsequent oligonucleotide cleaving steps. When ammonia which has been widely accepted as a safe reagent for DNA synthesis is utilized for cleaving, as taught by deBear et al., the cleavage time is as long as 24 hours at 65° C. In view of the growing trend to produce oligonucleotides as quickly as possible, this is an unacceptably long period of time. Decreasing the time required for cleaving the uridylyl from an oligonucleotide at the uridine 3' position typically uses a lead (II) ion catalyst system or the action of strong alkali hydroxides. Necessarily these processes require a separate isolation step to remove the lead ion. Additionally, when strong alkali bases are used in the cleaving processes, considerable side reactions in the form of cytosine deamination occur.

Recent improvements in the automation of simultaneous multiple oligonucleotide synthesis (whereby several oligonucleotides are synthesized simultaneously) further highlight the desirabilty of using a single universal solid support for the synthesis of oligonucleotides. Any requirement to dispense several different nucleoside preloaded solid supports on a DNA synthesizer clearly complicates the synthesis automation. Therefore, it would be highly desirable to have a universal solid support to synthesize any nucleic acid.

It is an object of the present invention to provide methods and compositions for use in the synthesis of oligonucleotides which ameliorate some of the problems encountered with the prior art methods and compositions. More particularly, it is an objective of the present invention to provide oligonucleotide synthesis reagents for synthesizing a variety of oligonucleotide independent of the initial nucleoside. It is another objective to provide oligonucleotide synthesis reagents suitable for using in connection with cleaving reagents which provide substantially decreased time for cleaving oligonucleotides from the solid support after synthesis.

### Summary of the Invention

The present invention provides methods and compositions which overcome prior art problems associated with the solid support synthesis of oligonucleotides. The compositions of the present invention provide universal solid support reagents suitable for the efficient step-wise oligonucleotide synthesis and the subsequent fast cleavage and deprotection of the synthesized oligonucleotide. In combination with previously disclosed cleaving reagents, using the solid support reagents of the present invention results in oligonucleotides synthesized in a substantially reduced length of time.

More particularly, the present invention provides oligonucleotide synthesis reagents having the following general formula: 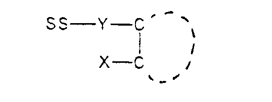 wherein SS is a solid support; Y is selected from the group consisting of esters, amides and thiol esters and X is selected from the group consisting of OH, SH, NH₂, and removable protecting groups; and the dotted lines represent an aromatic ring system having at least one aromatic ring, X and Y being vicinally positioned at adjacent carbon atoms of the at least one aromatic right .

Advantageously, these reagents are useful as universal solid supports in the synthesis of oligonucleotides wherein the synthesis takes place at the hydroxy, sulfhydryl, or amino functionality (X) vicinal to the ester, amide or thiol ester linkage. The ester, amide or thiol ester linkage Y is formed by reacting an hydroxy, thiol or amino aromatic substituent on the aromatic ring with a suitable acid moiety attached directly on the solid support or through a linking moiety, thereby linking the aromatic functionality to the solid support. Surprisingly, it has been determined that the post synthesis release of oligonucleotides synthesized using the oligonucleotide synthesis reagents of the present invention and the cleaving methods and reagents described in copending application S/N 08/636,113 filed April 22, 1996 invention is substantially faster than the release of oligonucleotides utilizing prior art solid support reagents. The newly synthesized oligonucleotides are released at the vicinal group position of the aromatic ring moiety in a reaction which involves nucleophilic attack at the ester or amide linkage followed by attack at the phosphate linkage between the initial nucleotide of the oligonucleotide and the aromatic compound. Methods for cleaving oligonucleotides synthesized in accordance with the methods of the present invention involve contacting a solid support bearing an oligonucleotide with a cleaving reagent which comprises a mixture of a first compound which includes methylamine and/or ammonium hydroxide and a second compound which can be a secondary amine and/or a tertiary amine. Preferably, the cleaving reagent is in the range of about 1:9 (v/v) of 40 wt% aqueous methylamine:23 - 25 wt% aqueous trimethyl amine to about 9:1 (v/v) 40 wt% aqueous methylamine:23 - 25 wt% aqueous trimethyl amine.

These and other advantages will become evident to those skilled in the art upon a more thorough understanding of the present invention as described in the following more detailed description of the invention.

### Detailed Description of the Invention

The present invention provides improvements in compositions and methods for solid support based oligonucleotide synthesis. More particularly, the present invention provides oligonucleotide synthesis reagents in which an aromatic ring moiety is linked to a solid support via an ester or amide linkage. The ring moiety has vicinally positioned functionalities, one of which is the site for the step wise synthesis of oligonucleotides and the other of which forms part of a linkage to the solid support. The linkage is preferably an ester or amide, but can also be a carbamate or other labile functionality. The site of the oligonucleotide synthesis is a hydroxy, a functional hydroxy equivalent such as sulfhydryl or amino, or protected hydroxyl or protected hydroxyl equivalent. In oligonucleotide synthesis procedures, the protected hydroxyl or hydroxyl equivalent is deprotected just prior to adding the initial nucleotide in the synthesis. The oligonucleotide synthesis reagent of the present invention is a universal solid support in that a single oligonucleotide reagent is useful for the synthesis of any oligonucleotide having any initial nucleoside, thus precluding the need to maintain a variety of different suitably derivatized solid supports.

Post synthesis, to cleave or remove the synthesized oligonucleotide from the solid support and release it from the aromatic ring moiety, a cleaving reagent hydrolyzes the functionality linking the ring moiety to the solid support, the resulting hydroxyl or hydroxyl equivalent then makes an intramolecular attack on the adjacent phosphate group of the first nucleotide of the oligonucleotide chain, forming a cyclic phosphate. This results in the release of the oligonucleotide molecule from the solid support. Whatever nucleoside is first added to the solid support during the oligonucleotide synthesis becomes the 3'-end terminal nucleoside or the 5'-end terminal of the synthesized molecule (when the oligonucleotide is synthesized in the conventional 3' to 5' direction and the 5' to 3' direction, respectively).

More particularly, the present invention provides an oligonucleotide synthesis reagent having the following general formula: 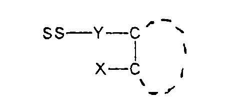 wherein SS is a solid support; Y from the group consisting of esters, amides, and thiol esters, and X is selected from the group consisting of -OH, -SH, -NH₂, and protected -OH,-SH, and NH₂; and the dotted lines represent carbon atoms forming a monocyclic or polycyclic aromatic ring system, X and Y being vicinally positioned with respect to each other at adjacent carbon atoms of the at least one aromatic ring. For reasons described below, vicinal groups X and Y are most effective when they are positioned so that they are on the same conformational plane. Since aromatic ring substitutions exist on the same plane, aromatic rings, as compared with alkyl rings where cis and trans conformations can exist, are preferred.

Those skilled in the art will appreciate that because of known protecting groups suitable for their protection, X is preferably O (oxygen). However, it will be apparent to those skilled in the art that utilizing NH and S in such positions is within the scope of the present invention. That is, hydrogen protecting groups for sulfhydryl and amino hydrogens can be removed and the initial nucleoside added to the sulfhydryl or amino group in a manner similar to that of addition to the hydroxy functionality.

The protecting group associated with X is suitably any protecting group which is easily removed using deprotection chemistries so that the once released or deprotected the group is available as the site for the introduction of a first nucleoside during the initiation of oligonucleotide synthesis. For purposes of the present invention, the 4,4'-dimethoxytrityl (DMT) group is particularly preferred. Other suitable groups include, but are not limited to, the following: 4,4',4"-tris- (benzyloxy)trityl (TBTr); 4,4',4"-tris- (4,5-dichlorophthalimido)trityl (CPTr); 4,4',4"-tris(levulinyloxy)trityl (TLTr); 3-(imidazolylmethyl)-4,4'-dimethoxytrityl (IDTr); pixyl (9-phenylxanthen-9-yl); 9-(p-methoxyphenyl)xanthen-9-yl (Mox); 4-decyloxytrityl (C₁₀Tr); 4-hexadecyloxytrityl (C₁₆Tr); 9-(4-octadecyloxyphenyl)xanthene-9-yl (C₁₈Px); 1,1-bis-(4-methoxyphenyl)-1'-pyrenyl methyl (BMPM) ; p-phenylazophenyloxycarbonyl (PAPoc); 9-fluorenylmethoxycarbonyl (Fmoc); 2,4-dinitrophenylethoxycarb onyl (DNPEoc);4-(methylthiomethoxy)butyryl (MTMB); 2-(methylthiomethoxymethyl)-benzoyl (MTMT); 2-(isopropylthiomethoxymethyl)benzoyl (PTMT); 2-(2,4-dinitrobenzenesulphenyloxymethyl)benzoyl (DNBSB); and levulinyl groups. These and other suitable protecting groups are described in detail in Beaucage, S. L. and Iyer, R. P. Tetrahedron 48, 2223-2311 (1992), the entire disclosure of which is hereby incorporated by reference.

As mentioned above, Y can be an amide, a thiol ester, a hydroxyl ester, or a functionality suitably labile under cleaving conditions utilizing ammonia and/or aliphatic amines. Thus, amides, thiol esters, and hydroxyl esters, respectively, provide oligonucleotide reagents having the following general structures: 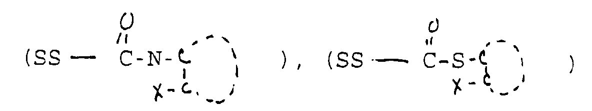 or a hydroxyl ester 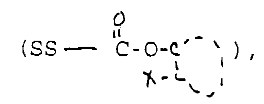 where X and SS are as further described herein. Because hydroxyl esters are easily formed and because dihydroxy vicinally substituted aromatic systems are readily available for forming hydroxyl esters, Y is preferably a hydroxyl ester for its ease in handling. Aromatic systems (represented by the dotted lines) suitable in the practice of the present invention include but are not limited to anthracene, phenanthracene, benzanthracene, coumarin, etc. Those skilled in the art will recognize that many polycyclic aromatic systems have applicability in the practice of the present invention.

As will be evident from the examples described herein the most preferred oligonucleotide synthesis reagents of the present invention have the following structures:

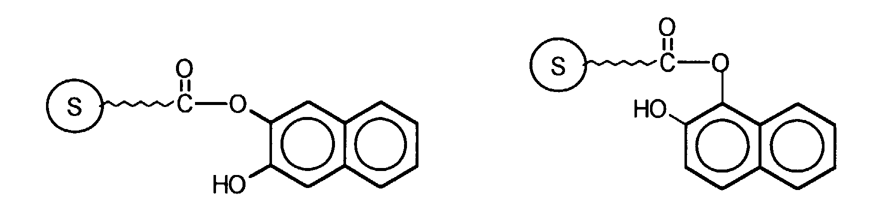

As would be readily appreciated by those working in the field, a wide variety of solid supports are conventional for use in oligonucleotide synthesis may be employed. The solid support comprises a functional group for attachment of a suitable ester linkage or amide linkage thereto by routine methods; common functional groups include hydroxyl, sulfhydryl and amino. The following exemplary supports have been employed: Controlled pore glass (CPG) [Pon, R. T. et al., Biotechniques 6, 768 (1988); Adams, S. P. et al., J. Amer. Chem. Soc. 105, 661 (1983)]; Fractogel [sold by E. Merck, Darmstadt, Germany, under the name "Fractogel" and by TosoHaas, Philadelphia, Pennsylvania, under the name "Toyopearl"; Reddy, M.P. et al., Tetrahedron Letters 35, 5771-5774 (1994)]; and tentagel [available from Rapp Polymere, Tubingen, Germany; Andrus et al., Tetrahedron Letters 34, 3373-3376 (1993)]. It will be understood by those skilled in the art that the solid support can be derivatized with a spacer linkage having a suitable functional group for forming the linkage to the appropriate vicinal reactive group. Such spacer linkages are favored by some because they can act as a leash in distancing the solid support from the reactive site on the aromatic ring. When a leash type linker is desired, the leash can be formed at the same time the synthesis reagent is formed. For example, an amino functionalized solid support such as CPG-NH₂ can be reacted with succinic anhydride to form a four carbon leash attached to CPG with an amide functionality and having a carboxylic acid functionality available for reacting with NH₂, SH or OH on a reagent of the present invention.

Other supports conventionally used in DNA synthesis [for example, as described in, e.g., Gait, M. J., Oligonucleotide Synthesis: A Practical Approach, IRL Press (1984) and U.S. Patent No. 4,923,901] would also be suitable for use in accordance with the present invention. Solid supports are suitably useful in a resinous or particulate form, other preformed shapes of any size, fibers, films etc.

Preparing the oligonucleotide synthesis reagents of the present invention can be carried out using conventional synthesis procedures. For example, solid supports having suitable surface reactive groups or reactive groups at the end of a leash or linker attached to the solid support (i.e. carboxylic acid moieties) can be reacted with a monocyclic or polycyclic aromatic system having vicinal NH2, OH, or SH substituents. The reactions conditions and reagents utilized are such that the aromatic system becomes linked to the solid support. Those skilled in the art will appreciate that the reaction linking a solid support to the aromatic ring results in attachment at one of the vicinal sites. This is due to the steric hindrance provided by the first attached solid support which blocks or impedes the attachment of a second solid support. It will also be appreciated that in the case of some vicinally substituted aromatic ring systems, a mixture of oligonucleotide reagents are formed. For example, 1,2 dihydroxynaphthalene reacted with a succinylated solid support will attached the solid support at the 1 position or the 2 position.

Advantageously, the oligonucleotide synthesis reagent of the present invention can be used in any oligonucleotide synthesis method capable of utilizing an unprotected or protected -OH, -SH, or -NH₂ including those methods using phosphoramidite reagents, the most widely used coupling chemistry for synthesis of oligonucleotides. However, other coupling chemistries are equally suitable for use, such as H-phosphonate chemistry [U.S. Patent No. 4,959,463; Froehler, B. C. et al., Nucleic Acids Research 14, 5399 (1986)] and triester chemistry [Stec, W. J. et al, Tetrahedron Letters 26, 2191 (1985); Gallo, K. O. et al, Nucleic Acids Research 14, 7406 (1986); Gait, M. J., Oligonucleotide Synthesis: A Practical Approach, IRL Press (1984)]. Thus, various kinds of nucleic acids may be employed in synthesis of oligonucleotides. The products which may be synthesized include DNA, RNA, oligonucleoside methylphosphonates [Agarwal, S. & Goodchild, J., Tetrahedron Letters 28, 3539 (1987)], oligonucleoside phosphorothioates [Beaucage, S.L. et al., J. Am. Chem. Soc. 112, 1253 (1990)], oligonucleoside phosphorodithioates [Dahl, O. & Bjergade, K., Nucleic Acids Research 19, 5843 (1991)], circular oligonucleotides [Kool, E. T. & Wang, S., Nucleic Acids Research 22, 2326 (1994); Kool, E. T. et al., J. Amer. Chem. Soc. 115, 360 (1993)], 2'-OMe RNA [Sproat, B. S. et al., Nucleic Acids Research 18, 41 (1989)], and products containing peptide nucleic acids [Nielsen, P. E. et al., Bioconjugate Chem. 5, 3 (1994)] or morpholine type backbone modified nucleic acids [Stirchak et al., Nucleic Acids Research 17 6129 (1989); U.S. Patent No. 5,034,506].

Advantageously, oligonucleotides synthesized utilizing the reagents of the present invention can be cleaved from the solid support to which they have been synthesized utilizing cleaving reagents and methods described in the above-identified copending application. Thus, cleaving oligonucleotides synthesized in accordance with the present invention involves contacting the solid support bearing an oligonucleotide with a cleaving reagent which includes a mixture of an amine selected from the group consisting of tertiary amines and secondary amines and a base selected from the group consisting of ammonium hydroxide and a primary amine.

A most preferred cleaving reagent is a solution of trimethylamine and methylamine. Additional secondary and tertiary amines suitable for cleaving oligonucleotides synthesized using the reagents of the invention include a variety of amines (bases with higher pKa's) such as triethylamine, n-propylamine, diisopropylamine, diisopropylethylamine, dimethylamine, diethylamine, piperidine, N-methylpiperidine and N-methylpyrrolidine. Preferred tertiary amines include trimethylamine, triethylamine, N-methylpyrrolidine, and diisopropylethylamine. As is demonstrated below the amount of ammonium hydroxide or primary amine and the amount of secondary amine or tertiary amine in the cleaving reagent can vary considerably. For example, in preferred cleaving reagents a ratio of methylamine or ammonium hydroxide to a secondary amine or tertiary amine is within the range of about 1:99 to about 99:1. Preferred concentration ratios and constituents are at least 9 parts of an aqueous methylamine solution to one part organic base having a basicity greater than ammonia. The most preferred cleaving reagent is 1 part of 40 wt% aqueous methylamine to 1 part of 23 - 25 wt% trimethylamine. (Those skilled in the art recognize that methylamine and trimethylamine are gases at standard temperature and pressure and their typical availability is in the form of an aqueous solution. Typically aqueous methylamine is 40 wt% methylamine and typical concentrations of trimethylamine are in the range of 23 - 25 wt%. As used in the context of the present invention, reference to a 1:1 v/v solution of methylamine and trimethylamine refers to a 1:1 v/v of a 40 wt% methylamine and about 24 wt% solution of trimethylamine.)

As mentioned briefly above, cleaving synthesized oligonucleotides from the solid support involves an intramolecular attack on the phosphate of the first nucleotides with the subsequent release of the synthesized oligonucleotide.

As will be appreciated by those skilled in the art, oligonucleotides prepared on the synthesis reagents of the present invention are more readily cleaved and removed from the solid support and the aromatic ring in part because of the ease of forming the phenoxide ion. Additionally, those skilled in the art will recognize that the phenoxide ion more readily attacks the adjacent phosphorous containing functionality. Another factor which contributes to the more desirable cleaving kinetics is the spatial geometry of the aromatic ring and the spatial geometry of the ring's substitutions. Because the ring is planar and its substitutions are planar, intramolecular attack between the ring's substitutions are sterically much more favorable.

For the most preferred embodiments of the present invention, the following generally illustrates preparing the oligonucleotide synthesis reagent, and subsequently cleaving the synthesized oligonucleotide from the solid support and aromatic ring structure.

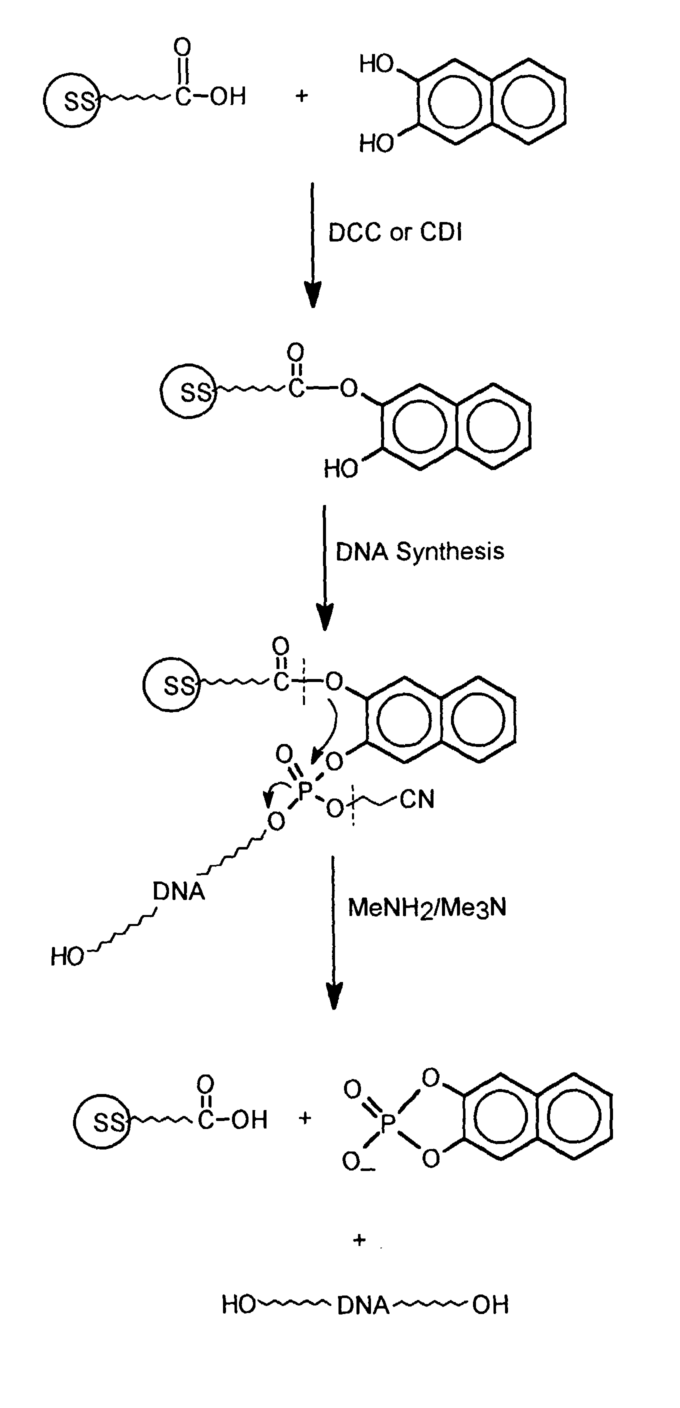

The invention may be better understood with reference to the accompanying examples, which are provided for purposes of illustration only and should not be construed as in any sense limiting the scope of the present invention as defined in the claims appended hereto.

### Examples

### Example 1

The following illustrates a method for preparing a solid support oligonucleotide synthesis reagent of the present invention and according to the following general synthetic procedures:

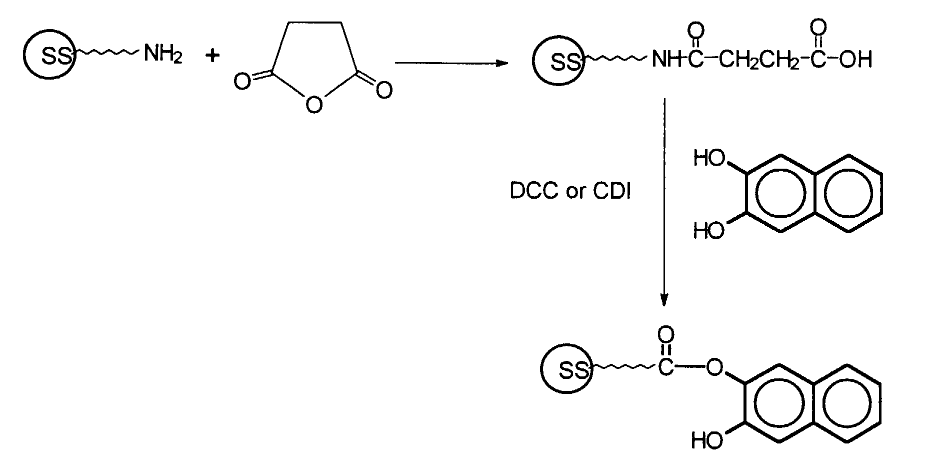

One (1) gram of aminated controlled pore glass (CPG-NH₂) obtained from CPG Inc., Lincoln Park, New Jersey, was added to a solution of 0.5 g (5mmol) of succinic anhydride and 425 mL (533 mmol) of 1-methylimidazole in 5 mL of dry pyridine. This mixture was shaken for 24 hours at room temperature. Then the succinylated CPG beads were removed from the solution by filtration, washed four times with 5 mL of pyridine and then four times with 5 mL of diethylether, and dried under vacuum. Next, the dried succinylated CPG beads were suspended in 5 mL of dry pyridine containing 167 mg (1.04 mmol) of 2,3-dihydroxynaphthalene, 100 mg (0.819 mmol) of DMAP, and 800 mg (3.88 mmol) of DCC. This suspension was shaken at room temperature for 48 hours after which 600 mg (4.31 mmol) of p-nitrophenol was added and the shaking was continued for an additional 24 hours. This reaction was quenched by shaking the reaction mixture for 3 hours in 334 µL (3.83 mmol) of morpholine. After shaking an additional 3 hours, the treated CPG beads were removed by filtration, washed four times with 5 mL of methanol, washed four times with 5 mL diethylether and then dried under vacuum.

The degree to which the dihydroxynaphthalene reacted with the succinylated CPG or the degree of naphthalene loading was determined by coupling thymidine onto an accurately determined weight of the derivatized CPG with DMT-on. Then during manual detritylation with 3% trichloroacetic acid in dichloromethane the amount of released DMT was determined using absorbance at 500 nm of the dimethoxytrityl cation released. The results indicated that the naphthalene coupled to the succinylated CPG at 16 µmol/g of CPG.

### Example 2

The following illustrates an alternative method for preparing an oligonucleotide synthesis reagent of the present invention.

Aminated controlled pore glass (CPG-NH₂) was succinylated according to the succinylation procedure described in Example 1. After washing the succinylated CPG beads in pyridine and diethylether and drying in vacuum the beads were suspended in 10 mLs of dry dichloromethane containing 1.62 g (10 mmol) of 1,1'-carbonyldiimidazole (CDI). This suspension was shaken at room temperature for 24 hours. Then the treated CPG beads were filtered, washed 4 times with 10 mL of dry dichloromethane and suspended in 10 mL of dry dichloromethane. After adding 1.62 g (10 mmol) of 2,3-dihydroxynaphthalene, the suspension was shaken at room temperature for another 24 hours. After adding 2 mL of anhydrous methanol to the suspension it was shaken for an additional 2 hours. The treated glass beads were then removed by filtration, washed four times with 5 mL methanol, washed 4 times with 5 mL diethylether, and dried under vacuum.

The degree to which naphthalene was loaded onto the succinylated CPG beads was determined in the same manner as described in Example 1. The results indicated that the naphthalene loading was 30 µmol/g of CPG.

### Example 3

The following describes the synthesis of oligonucleotides having the sequence 5'-GAT GCC AGT TCG GTC ATA CAC GTA GTA CTA CGA CX-3'(where X= T,C,G, or A) SEQ ID NO:1) on the solid support synthesis reagent described in Example 1 according to the following general procedure:

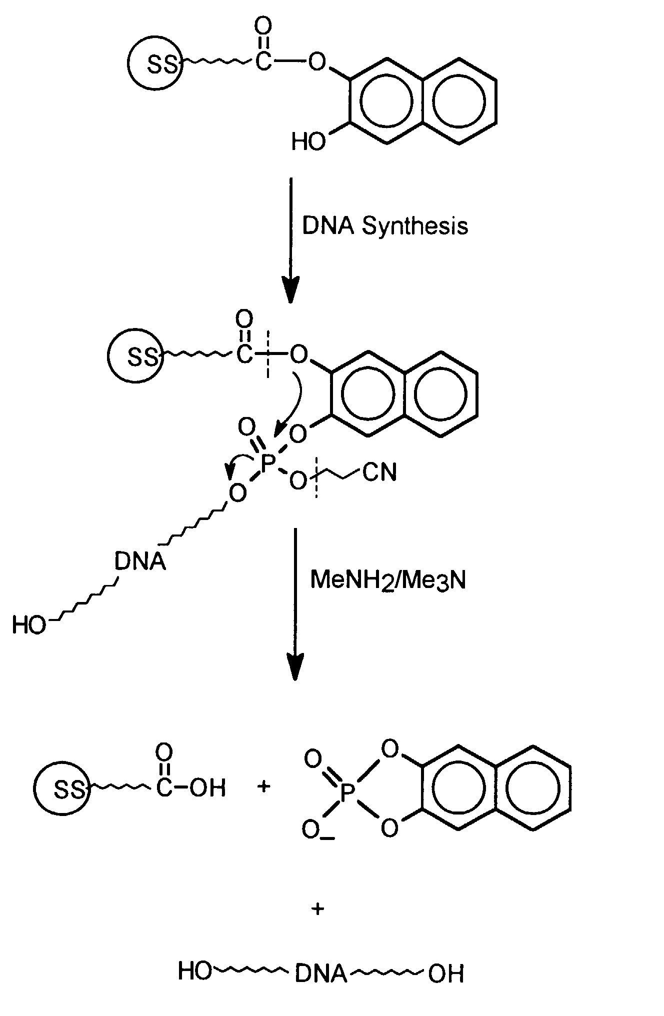

Four different oligonucleotides having the above identified sequence and differing only in the 3' terminus nucleotide were assembled on a Beckman Instruments Oligo 1000 DNA automated synthesizer using standard phosphoramidite chemistries. Manufacturer's directions were followed and manufacturer's reagents were utilized except that the solid support installed in the instrument was the dihydroxynaphthalene loaded CPG prepared as described in Example 1.

At the end of the synthesis and after removing the last trityl group, the synthesized oligonucleotide was cleaved from the solid support at the 3'terminal naphthalene moiety by contacting the solid support with a solution of CH3NH2 and (CH3)3N at room temperature for 5 minutes. The cleaving solution was 1:1 (v/v) solution of 40 wt% methylamine in water and 24 wt% trimethylamine in water.

After removing the cleaved oligonucleotide from the solid support the solution containing the cleaved oligonucleotide was heated at 65 °C for 2.5 hours to release the naphthalyl moiety from the oligonucleotide. Then the reagent was evaporated to dryness using a vacuum concentrator. The residue was dissolved in 400 µL of double distilled water and the mass of oligonucleotide was determined using the absorbance at 260 nm. The oligonucleotide products were then analyzed by performing capillary gel electrophoresis using a P/ACE automated CE system. The resulting electropherograms were compared with electropherograms obtained of the same sequence control oligonucleotides prepared using a prior art conventional CPG solid support. The results indicated a good quality product comparable to the control oligonucleotides.

### Example 4

In order to demonstrate the versatility of oligonucleotide solid support reagents of the present invention additional oligonucleotides were synthesized and analyzed by comparing their electropherograms with electropherograms of control oligonucleotides. The following oligonucleotides were synthesized using the synthesis procedure, automated instrumentation and cleaving methods described in Example 3. The solid support prepared in Examples 1 and 2 were utilized in these experiments.

A 17 mer having the sequence:

5' TGT AAA TGC CAT CGT CC 3' (SEQ ID NO:2);

Four (4) 10 mers having the sequence:

5' GTC GAC AGC X 3' (where X = A, T, G and C)
(SEQ ID NO:3)

An 101 mer having the sequence:

5' AAC GTC GGT AAC GTA CAC GGT AGC TAC GGA CAC
CGT GGC AAT ACG ACA GGT AAC CTG TGG AAC GTA CAC GGA AGA
GAC TAG GGA TGG GAG TAC GGA TCC GT 3'. (SEQ ID NO:4)

The results of comparing electropherograms of the oligonucleotides prepared using the oligonucleotide synthesis reagents of the present invention with electropherograms of the same oligonucleotide sequences prepared as controls using prior art conventional CPG solid support showed that the solid supports of the present invention provide comparable oligonucleotides.

### Example 5

The following demonstrates the successful synthesis of a 21 mer RNA on a oligonucleotide solid support synthesis reagent of the present invention. The sequence of the synthesized RNA is as follows:

5' CUG GAC ACU AGU CCG ACU GCU 3' (SEQ ID NO:5)

0.2 µmoles of the oligonucleotide synthesis reagent prepared in Example 2 (naphthalene loading of 30 µmoles/gm) was placed in an Oligo 1000 instrument column and the synthesis of the above identified RNA was programmed to proceed using standard Beckman reagents and instrument procedures. The coupling time was 12 minutes. Cleaving the synthesized RNA from the naphthalene moiety and the solid support was carried out using the above identified cleaving reagent CH₃NH₂/(CH₃)₃N (a 1:1 v/v solution of 40% aqueous CH₃NH₂ and 24% aqueous (CH₃)₃N). Reaction time was 3 hours at 65°C. The RNA 2' protecting group was removed using 1.0 M tetrabutylammonium fluoride in THF for 15 hours at room temperature. The oligonribonucleotide was desalted using Sep-Pak (C18 cartridges from Millipore).

The RNA samples were lyophilized and analyzed on a Beckman P/ACE automated capillary electrophoresis. The U100P Urea Gel column available from Beckman Instruments was used with a running buffer of tris-borate and 7M Urea. The electropherograms indicated a 24.95 minute retention time for the synthesized 21 mer.

### Example 6

The following example demonstrates the successful synthesis of a 25 mer phosphorothioate utilizing an oligonucleotide synthesis reagent of the present invention. The 25 mer had the following sequence:

5' AGT CAG TCA GTC AGT CAG TCA GTC T 3' (SEQ ID NO:6)

The oligonucleotide synthesis reagent prepared in Example 2 was loaded into an Oligo 1000 column and using a 0.2 µmole scale the above phosphorothioate oligonucleotide was synthesized using manufacturer's reagents and procedures. At the completion of the synthesis the 25 mer was cleaved from the CPG and the naphthalene moiety utilizing the cleaving reagent of Example 3 and a 3 hour 65°C cleaving conditions. The sample was analyzed by HPLC and capillary electrophoresis and retention times were compared with the phosphorthioates synthesized under the same conditions except that prior art conventional CPG supports were utilized.

### Example 7

The following demonstrates the successful preparation and use of an oligonucleotide synthesis reagent of the present invention in which the aromatic ring structure is catechol.

Succinylated aminated controlled pore glass was prepared as described in Example 1. Two hundred fifty mg (250 mg) of the dried succinylated CPG were suspended in 5 mL of dry pyridine containing 29 mg (0.26 mmol) of catechol, 25 mg (0.20 mmol) of DMAP, and 200 mg (0.969 mmol) of DCC. This suspension was shaken at room temperature for 48 hours after which 150 mg (1.08 mmol) of p-nitrophenol was added and the shaking was continued for an additional 24 hours. This reaction was quenched by shaking the reaction mixture for 3 hours with 84 µL of morpholine. The treated CPG beads were removed by filtration, washed four times with 5 mL of methanol, washed four times with 5 mL diethylether and then dried under vacuum. The degree to which the catechol was attached to the succinylated CPG was determined to be 16.3 µmol/g.

The following sequences were synthesized onto the just described oligonucleotides synthesis reagent according to the synthesis method described in Example 3. 
5' TGT AAA TGC CAT CGT CT 3' (SEQ ID NO:2)

5' GAT GCC AGT TCG GTC ATA CAC GTA GTA CTA CGA CGA CT
3' (SEQ ID NO:1)
 The synthesized oligomers were deprotected and removed from the solid support using a cleaving solution of 1:1 (v/v) 40 wt% aqueous methylamine and 24 wt% aqueous trimethyl amine at 65°C overnight.

The quality of the 35 mer (SEQ ID NO:1) and the 17 mer (SEQ ID NO:2) was confirmed using capillary gel electrophoresis. Additionally, the 17 mer was analyzed using high performance anion exchange chromatography.

### Example 8

The following example demonstrates the preparation and use of an oligonucleotide synthesis reagent of the present invention in which the aromatic ring structure is 1,2 dihydroxynaphthalene

Succinylated aminated controlled pore glass was prepared as described in Example 1. Two hundred fifty mg (250 mg) of the dried succinylated CPG were suspended in 5 mL of dry pyridine containing 42 mg (0.26 mmol) of 1,2 dihydroxynaphthalene, 26 mg (0.21 mmol) of DMAP, and 950 mg (4.60 mmol) of DCC. This suspension was shaken at room temperature for 48 hours after which 153 mg (1.10 mmol) of p-nitrophenol was added and the shaking was continued for an additional 24 hours. This reaction was quenched by shaking the reaction mixture for 3 hours with 84 µL of morpholine. The treated CPG beads were removed by filtration, washed four times with 5 mL of methanol, washed four times with 5 mL diethylether and then dried under vacuum. The degree to which the 1,2 dihydroxynaphthalene was attached to the succinylated CPG was determined to be 19.7 µmol/g using the procedure described in example 1.

A T₅ and SEQ ID NO:1 were synthesized on to the just described oligonucleotide synthesis reagent according to the procedure described in Example 3. Following the synthesis the oligonucleotides were cleaved and deprotected successfully as described in Example 7.

### Example 9

The following example demonstrates the successful use of oligonucleotides prepared utilizing reagents of the present invention as primers in standard PCR reactions.

An 18 mer and 22 mer were prepared on an Oligo 1000 DNA synthesizer utilizing the synthesis reagent of Example 2. The same 18 mer and 22 mer were also prepared on an Oligo 1000 utilizing a prior art conventional CPG solid support. Each of the oligonucleotides were Sep Pak purified. The 18 mer and 22 mer had the following sequences:

5' CGC CAG GGT TTT CCC AGT 3' (SEQ ID NO:7)

5' TTC TGG CGT ACC GTT CCT GTC T 3' (SEQ ID NO:8)

Each of the above identified sequences was utilized as a primer in standard PCR reactions using a M13mpl8RFlDNA template purchased from New England Biolabs cat. #400-18). The PCR procedure was that of Perkin-Elmer Cetus Gene Amp DNA Amplification Reagent Kit with Amplitaq Recombinant Taq DNA polymerase. The initial melting temperature was 95 °C for 7 minutes and 25 cycles were carried out using the Perkin-Elmer Cetus DNA Thermal Cycler with the following cycle profile:
#1 94°C
   0 min 1 sec
#2 94°C
   1 min 0 sec
#3 37°C
   0 min 1 sec
#4 37°C
   2 min 0 sec
#5 72°C
   0 min 1 sec
#6 72°C
   3 min 0 sec
Each 957 base pair PCR product was electrophoresed on a 1% agarose slab gel in TAE and stained with ethidium bromide. PCR products obtained using the synthesized oligonucleotide primers on conventional supports and reagent support of the present invention were identical. This demonstrates the successful use of PCR primers synthesized utilizing the oligonucleotide synthesis reagents of the present invention.

While the invention has been described in connection with specific embodiments thereof, it will be understood that it is capable of further modifications and this application is intended to cover any variations, uses, or adaptations of the invention following, in general, the principles of the invention and including such departures from the present disclosure as come within known or customary practice within the art to which the invention pertains and as may be applied to the essential features hereinbefore set forth and as follows in the scope of the appended claims.

## Claims

1. An oligonucleotide synthesis reagent comprising a composition having the formula: 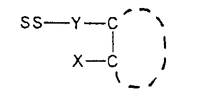 wherein SS is a solid support; Y is selected from the group consisting of ester, amide, and thiol ester groups; X is selected from the group consisting of -OH, -SH, -NH₂, and protected OH, -SH, and NH₂; and the dotted lines represent an aromatic ring system having at least one aromatic ring, X and Y being vicinally positioned with respect to each other at adjacent carbon atoms of the at least one aromatic ring.

2. The oligonucleotide reagent of claim 1 wherein said aromatic ring is selected from the group consisting of naphthyl and phenyl.

3. The oligonucleotide synthesis reagent of claim 1 wherein X is trityl.

4. The oligonucleotide synthesis reagent of claim 1 wherein the solid support is selected from the group consisting of controlled pore glass, copolymers of ethylene and acrylate, copolymers of ethylene and methacrylate, polystyrene, and nylon.

5. An oligonucleotide synthesis reagent comprising a compound selected from the group of formulas consisting of: 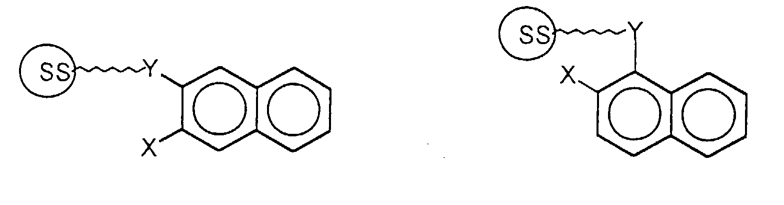 wherein SS is a solid support; Y is selected from the group consisting of esters, amides and thiol esters; and X is selected from the group consisting of -OH, -SH, -NH₂, and - protected OH, -SH, and NH₂.

6. The oligonucleotide synthesis reagent of claim 5 wherein the solid support is selected from the group consisting of controlled pore glass, copolymers of ethylene and acrylate, copolymers of ethylene and methacrylate, polystyrene, polypropylene and nylon.

7. The oligonucleotide synthesis reagent of claim 5 wherein the solid support includes a linker functionality linking the solid support to Y.

8. The oligonucleotide synthesis reagent of claim 5 wherein Y is a carboxylic acid ester linkage and X is trityl.

9. The oligonucleotide synthesis reagent of claim 9 wherein the ring moiety is 2,3 dihydroxynaphthalene.

10. A process for synthesizing an oligonucleotide, said process comprising the steps:
providing an oligonucleotide synthesis reagent having the formula: 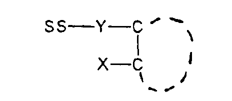 wherein SS is a solid support; Y is selected from the group consisting of esters, and amides, and thiol esters; X is selected from the group consisting of -OH, -SH, -NH₂, and - protected OH, -SH, and NH₂; and the dotted lines represent an aromatic ring system having at least one aromatic ring, X and Y being vicinally positioned with respect to each other at adjacent carbon atoms of the at least one aromatic ring; and
sequentially coupling protected nucleoside monomer units to provide an oligonucleotide.

11. The process for synthesizing an oligonucleotide of claim 10 wherein said synthesis reagent is selected from the group consisting of: 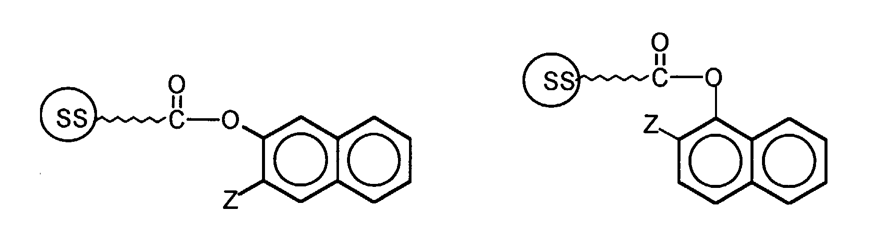 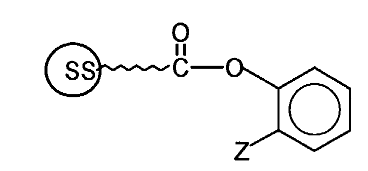 where SS is a solid support and Z is selected from the group consisting of -OH, -SH, - NH₂, and protected -OH, -SH, and NH₂.

12. The process for synthesizing an oligonucleotide of claim 10 wherein the solid support is selected from the group consisting of controlled pore glass, copolymers of ethylene and acrylate, copolymers of ethylene and methacrylate, polystyrene, polypropylene and nylon.

13. The process for synthesizing an oligonucleotide of claim 10 wherein the solid support further includes a linker functionality linking the solid to Y.

14. The oligonucleotide synthesis reagent of claim 10 wherein X trityl and Y is a carboxylic acid ester.

15. The process for synthesizing an oligonucleotide of claim 10 wherein the ring moiety is 2,3 dihydroxynaphthalene.

16. The process of claim 10 wherein said synthesis reagent has the following structure: 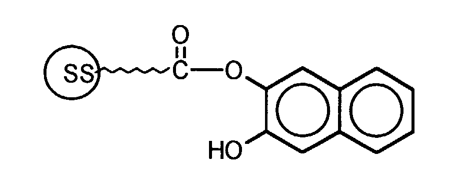 where SS a solid support

17. The process for synthesizing an oligonucleotide of claim 10 further including the step of cleaving and deprotecting a synthesized oligonucleotide by contacting the oligonucleotide synthesis reagent with a solution of aqueous methylamine and trimethylamine.


SEQUENCE LISTING

(1) GENERAL INFORMATION:

(i) APPLICANT: BECKMAN INSTRUMENTS, INC.

(ii) TITLE OF INVENTION: UNIVERSAL SOLID SUPPORT
OLIGONUCLEOTIDE SYNTHESIS
REAGENTS

(iii) NUMBER OF SEQUENCES: 8

(iv) CORRESPONDENCE ADDRESS:
(A) ADDRESSEE: BECKMAN INSTRUMENTS, INC.
(B) STREET: 2500 HARBOR BOULEVARD
(C) CITY: FULLERTON
(D) STATE: CA
(E) COUNTRY: USA
(F) ZIP: 92834-3100

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25

(vi) CURRENT APPLICATION DATA:
(A) APPLICATION NUMBER:
(B) FILING DATE:
(C) CLASSIFICATION:

(vii) PRIOR APPLICATION DATA:
(A) APPLICATION NUMBER: 08/714,841
(B) FILING DATE: September 6, 1996
(C) CLASSIFICATION:

(viii) ATTORNEY/AGENT INFORMATION:
(A) NAME: HARDER, P. R.
(B) REGISTRATION NUMBER: 20,022
(C) REFERENCE/DOCKET NUMBER: 045D-1530

(ix) TELECOMMUNICATION INFORMATION:
(A) TELEPHONE: (714) 773-6929
(B) TELEFAX: (714) 773-7936

(2) INFORMATION FOR SEQ ID NO:1:

(i) SEQUENCE CHARACTERISTICS:
(A) LENGTH: 35 bases
(B) TYPE: nucleic acid
(C) STRANDEDNESS: single
(D) TOPOLOGY: linear

(ii) MOLECULE TYPE: DNA (genomic)

(iii) HYPOTHETICAL: YES

(iv) ANTI-SENSE: NO

(xi) SEQUENCE DESCRIPTION: SEQ ID NO:1:

GATGCCAGTT  CGGTCATACA  CGTAGTACTA  CGACN  35

(2) INFORMATION FOR SEQ ID NO:2:

(i) SEQUENCE CHARACTERISTICS:
(A) LENGTH: 17 bases
(B) TYPE: nucleic acid
(C) STRANDEDNESS: single
(D) TOPOLOGY: linear

(ii) MOLECULE TYPE: DNA (genomic)

(iii) HYPOTHETICAL: YES

(iv) ANTI-SENSE: NO

(xi) SEQUENCE DESCRIPTION: SEQ ID NO:2:

TGTAAATGCC  ATCGTCC  17

(2) INFORMATION FOR SEQ ID NO:3:

(i) SEQUENCE CHARACTERISTICS:
(A) LENGTH: 10 bases
(B) TYPE: nucleic acid

(C) STRANDEDNESS: single
(D) TOPOLOGY: linear

(ii) MOLECULE TYPE: DNA (genomic)

(iii) HYPOTHETICAL: YES

(iv) ANTI-SENSE: NO

(xi) SEQUENCE DESCRIPTION: SEQ ID NO:3:

GTCGACAGCN 10

(2) INFORMATION FOR SEQ ID NO:4:

(i) SEQUENCE CHARACTERISTICS:
(A) LENGTH: 10 1base pairs
(B) TYPE: nucleic acid
(C) STRANDEDNESS: single
(D) TOPOLOGY: linear

(ii) MOLECULE TYPE: RNA (genomic)

(iii) HYPOTHETICAL: YES

(iv) ANTI-SENSE: NO

(xi) SEQUENCE DESCRIPTION: SEQ ID NO:4:

AACGTCGGTA ACGTACACGG TAGCTACGGA CACCGTGGCA ATACGACAGG
TAACCTGTGG AACGTACACG GAAGAGACTA GGGATGGGAG TACGGATCCG T 101

(2) INFORMATION FOR SEQ ID NO:5:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: RNA (genomic)

(iii) HYPOTHETICAL: YES

(iv) ANTI-SENSE: NO

(xi) SEQUENCE DESCRIPTION: SEQ ID NO:5:

CUGGACACUA GUCCGACUGC U 21

(2) INFORMATION FOR SEQ ID NO:6:

(i) SEQUENCE CHARACTERISTICS:
(A) LENGTH: 25 bases
(B) TYPE: nucleic acid
(C) STRANDEDNESS: single
(D) TOPOLOGY: linear

(ii) MOLECULE TYPE: DNA (genomic)

(iii) HYPOTHETICAL: YES

(iv) ANTI-SENSE: YES

(xi) SEQUENCE DESCRIPTION: SEQ ID NO:6:

AGTCAGTCAG TCAGTCAGTC AGTCT 25

(2) INFORMATION FOR SEQ ID NO:7:

(i) SEQUENCE CHARACTERISTICS:
(A) LENGTH: 18 Bases
(B) TYPE: nucleic acid
(C) STRANDEDNESS: single
(D) TOPOLOGY: linear

(ii) MOLECULE TYPE: DNA (genomic)

(iii) HYPOTHETICAL: YES

(iv) ANTI-SENSE: NO

(xi) SEQUENCE DESCRIPTION: SEQ ID NO:7:

CGCCAGGGTT TTCCCAGT 18

(2) INFORMATION FOR SEQ ID NO:8:

(i) SEQUENCE CHARACTERISTICS:
(A) LENGTH: 22 bases
(B) TYPE: nucleic acid
(C) STRANDEDNESS: single
(D) TOPOLOGY: linear

(ii) MOLECULE TYPE: DNA (genomic)

(iii) HYPOTHETICAL: YES

(iv) ANTI-SENSE: YES

(xi) SEQUENCE DESCRIPTION: SEQ ID NO:8:

TTCTGGCGTA CCGTTCCTGT CT 22